Europäisches Patentamt

European Patent Office

Office européen des brevets

(11) Publication number: **0 528 458 A1**

# EUROPEAN PATENT APPLICATION

(21) Application number: **92202024.3**

(22) Date of filing: **03.07.92**

(51) Int. Cl.5: **A61K 7/16**

(30) Priority: **09.08.91 GB 9117315**

(43) Date of publication of application:
**24.02.93 Bulletin 93/08**

(84) Designated Contracting States:
**AT BE CH DE DK ES FR GB GR IT LI NL PT SE**

(71) Applicant: **UNILEVER N.V.**
**Weena 455**
**NL-3013 AL Rotterdam(NL)**

(84) **BE CH DE DK ES FR GR IT LI NL PT SE AT**

(71) Applicant: **UNILEVER PLC**
**Unilever House Blackfriars P.O. Box 68**
**London EC4P 4BO(GB)**

(84) **GB**

(72) Inventor: **Burger, Allan Robert**
**Unilever Research U.S., Inc., 45 River Road**
**Edgewater, NJ 07020(US)**
Inventor: **Schick, Laura Ann**
**Unilever Research U.S., Inc., 45 River Road**
**Edgewater, NJ 07020(US)**

(74) Representative: **van Gent, Jan Paulus et al**
**Unilever N.V., Patent Division Postbus 137**
**NL-3130 AC Vlaardingen (NL)**

(54) **Method for controlling dental tartar using phosphopeptides and compositions for use therein.**

(57) A method and composition for the control of tartar and brushite formation in the mouth is disclosed based upon the use of mixtures of particular phosphopeptides with a water-soluble polyphosphate salt and/or a water-soluble zinc salt. The phosphopeptides comprise 5 to 30 aminoacids, and include the aminoacid sequence A-B-C-D-E, wherein A, B, C, D and E are each independently phosphoserine, phosphothreonine, phosphotyrosine, phosphohistidine, glutamate and aspartate. Mixtures of the phosphopeptides with alkalimetal pyrophosphates and/or zinc citrate have been found to be particularly useful for tartar control.

EP 0 528 458 A1

Rank Xerox (UK) Business Services
(3.10/3.5x/3.0.1)

## BACKGROUND OF THE INVENTION

### Field of the Invention

The present invention relates to a method of controlling dental tartar which comprises contacting the teeth with an oral composition which contains particular phosphopeptides and water-soluble polyphosphate salts and/or water-soluble zinc salts. It also relates to oral compositions for use in said method, said compositions comprising particular phosphopeptides and water-soluble polyphosphate salts and/or water-soluble zinc salts.

### Related Art

Tartar, also known as calculus, is a hard, mineralized deposit which forms on the surfaces of the lower front teeth and some upper molars. Mature tartar consists of an inorganic portion which is largely calcium phosphate, and an organic portion which contains desquamated epithelial cells, leukocytes, salivary sediment, food debris and various non-viable microorganisms. The calcium phosphate is deposited on the surfaces of the teeth in the pellicle and in the extracellular matrix of dental plaque in the form of crystals. Various forms of calcium phosphate crystals have been identified, the most difficult to remove and thermodynamically most stable form being the hydroxyapatite form. Mature tartar contains calcium phosphate largely arranged in this hydroxyapatite crystal lattice structure.

Another form of calcium phosphate is the thermodynamically at neutral pH unstable form called brushite; amorphous forms of calcium phosphates and brushite are believed to be the precursors of hydroxyapatite, the amorphous forms and brushite transforming into the hydroxyapatite form.

It would, therefore, be desirable to prevent or delay such amorphous forms of calcium phosphate and brushite from transforming into the hydroxyapatite form, and indeed the art has already recognized that agents which interfere with the formation of hydroxyapatite crystals can be effective anti-tartar agents. Thus, Parran, Jr. et al. have suggested in US 4,515,772, US 4,590,077 and US 4,684,518 the use of soluble inorganic alkalimetal pyrophosphates as tartar control agents in oral compositions. Other proposals include the use of polymeric materials such as polyacrylates (US 4,662,341; Benedict et al.) and linear anionic (co)-polymers such as polymethylvinylether/maleic anhydride copolymers (US 4,627,977, US 4,806,340, US 4,806,342, US 4,808,400, US 4,808,401; all by Gaffar et al.), with or without soluble inorganic pyrophosphate salts. Despite these many proposals, the need for an effective control of brushite and tartar formation still exists.

U.S. Patent 4,534,881 (Sikes et al.) discloses synthetically derived amino acid polymers for the inhibition or retardation of inorganic scaling, such as calcium carbonate deposition in pipes, boilers and the like. The synthetic polymers may contain phosphorylated amino acids.

U.S. Patent 4,866,161 (Sikes et al.) and U.S. Patent 4,868,287 (Sikes et al.) disclose a synthetic poly amino acid compound and a method of inhibiting tartar deposition on teeth by applying the synthetic poly amino acid compound, wherein the anionic amino acids are clustered on one end of the amino acid chain and the nonpolar amino acids are clustered on the other end. The anionic amino acids are independently selected from phosphoserine, phosphohomoserine, phosphotyrosine, phosphothreonine, glutamic acid and aspartic acid. Although the poly amino acids taught by Sikes et al. must be synthesized, Sikes et al. disclose that the presence of phosphoserine residues in natural proteins significantly enhances anti-tartar activity and illustrate that statherin (a natural salivary phosphoprotein) is an effective tartar inhibitor.

European Patent Application 0 391 629 (Sikes) discloses synthetic polypeptide materials having a formula $poly(X)_m(Y)_n$, where each X is independently aspartate, glutamate, glutamine, asparagine, or phosphoserine, each Y independently is a phosphorylated amino acid such as phosphoserine, phosphohomoserine, phosphotyrosine and phosphothreonine; m is 2 to 150, n is 1 to 3, and n + m is greater than or equal to 5. The '629 application discloses that mineralization inhibiting compositions may additionally contain other compounds which are known to inhibit mineralization, for example, zinc citrate.

The poly amino acids taught in all of the Sikes et al. references discussed above must be synthesized. While statherin, a naturally occurring salivary protein is mentioned by Sikes et al., it is hard to obtain a commercially sufficient supply of statherin. In the present invention phosphopeptides, which differ from the materials disclosed by Sikes et al. and also differ from statherin, can be obtained from naturally occurring materials, such as casein (a milk protein), yet, may be obtained in amounts sufficient to satisfy commercial demand.

It has now been found that certain phosphopeptides, which can be obtained from naturally occurring materials, in combination with a water-soluble polyphosphate salt and/or a water-soluble zinc salt provide for an effective control of tartar. Additionally, the compositions employed in the present invention have been found to possess superior brushite growth inhibitory activity. In this respect, it is observed, that in European Patent Applications 0,073,210 and 0,166,055 the use of certain proteins and peptides in oral compositions has been disclosed. These materials are included in these compositions as caries- and gingivitis-inhibiting agents. European Patent Application 0,268,663 discloses the use of the phosphopeptides employed in the present invention as anti-caries and anti-gingivitis agents. The '663 Application discloses an anti-caries composition containing a phosphopeptide and 0.1 mM zinc acetate. No mention is made in any of these European Patent Applications of any potential anti-tartar or anti-brushite efficacy of the disclosed compositions.

It is, therefore, an object of the present invention to provide an effective method for controlling tartar and brushite by treating the teeth with particular phosphopeptides in combination with a water-soluble polyphospate salt and/or a water-soluble zinc salt.

It is another object of the present invention to provide oral compositions comprising particular phosphopeptides and a water-soluble polyphosphate salt and/or a water-soluble zinc salt, suitable for use in a method for controlling tartar.

These and other objects of the present invention will become more clear from the detailed description and examples which follow.

SUMMARY OF THE INVENTION

The present invention embraces a method for controlling brushite and dental tartar. The method comprises treating the teeth with an oral composition which comprises a phosphopeptide or a salt thereof, the phosphopeptide having from 5 to 30 aminoacids including the aminoacid sequence A-B-C-D-E, wherein A, B, C, D and E are independently phosphoserine, phosphothreonine, phosphotyrosine, phosphohistidine, glutamate and aspartate, and a water-soluble polyphosphate salt and/or a water-soluble zinc salt, the phosphopeptide and the polyphosphate salt and/or the zinc salt being present in an effective amount to control the formation of tartar.

The present invention also encompasses an oral composition in the form of a liquid dentifrice, a mouthwash or a toothpaste, suitable for use in the method for controlling dental tartar, said composition comprising an effective amount of the above-identified phosphopeptides in combination with a water-soluble polyphosphate salt and/or a water-soluble zinc salt. The inventive compositions include the polyphosphate and/or the zinc salt in an effective amount to inhibit tartar formation.

DETAILED DESCRIPTION OF THE INVENTION

One of the essential elements in the method for controlling dental tartar according to the present invention is the use of the particular phosphopeptides or salts thereof. The phosphopeptides contain from 5 to 30 aminoacids including the sequence

A-B-C-D-E

where A, B, C, D and E are independently phosphoserine, phosphothreonine, phosphotyrosine, phosphohistidine, glutamate and aspartate.

Preferred phosphopeptides are those wherein A, B and C are independently phosphoserine, phosphothreonine, phosphotyrosine, and phosphohistidine and D and E are independently phosphoserine, phosphothreonine, glutamate and aspartate.

Particularly preferred phosphopetides are those where A, B and C are phosphoserine and D and E are glutamate.

A mixture of phosphopeptides and/or their salts may be used in the method of the present invention. In this instance it is preferred that those containing the preferred sequence A-B-C-D-E above predominate.

The phosphopeptides or mixture of phosphopeptides is preferably substantially pure at least to the extent of not containing unpalatable impurities.

The phosphopeptides identified as SEQ ID NO:1, SEQ ID NO:2, SEQ ID NO:3, SEQ ID NO:4, and SEQ ID NO:5 in the "Sequence Listing" hereinbelow have been found to be particularly useful in the compositions of the present invention.

The preferred phosphopeptide may be made synthetically by chemical synthesis or genetic engineering or can be extracted from naturally occurring material e.g. milk.

Because of cost considerations, it is currently more economical to extract the phosphopeptide from casein or caseinates and in particular from alpha-s casein or beta-casein. Additionally, there is an increasing consumer demand for products containing ingredients derived from naturally occurring materials. Thus, in the preferred embodiment of the invention, the phosphopeptide is extracted from the naturally occurring material.

Phosvitin may also be used as a source of phosphopeptides. Further, phosphoproteins in cereals, nuts and vegetables, particularly in bran husks or sheaths, may be used to produce phosphopeptides. In particular, rice, wheat, oat, barley or rye brans are suitable sources of phosphopeptides. Soybean and meat contain phosphoproteins which may also be of use in obtaining phosphopeptides.

Casein, in particular alpha-s casein or beta-casein, or salts thereof such as sodium caseinate contain polypeptides which can be cleaved to simpler peptides. Such cleavage may be effected by digestion, such digestion may be chemical, or enzymatic with proteolytic enzymes.

It is preferred to digest casein with a proteolytic enzyme such as trypsin, pepsin, chymotrypsin, papain, thermolysin or pronase. Trypsin is the preferred enzyme. The digested casein is then fractionated into phosphopeptides containing the sequence A-B-C-D-E, and other peptides. Particularly preferred in the compositions of the present invention are the phosphopeptides of SEQ ID NO:1 and SEQ ID NO:2 mentioned above, as well as mixtures of SEQ ID NO:1 and SEQ ID NO:2, and mixtures rich in phosphopetides having the sequence of SEQ ID NO:1 and/or SEQ ID NO:2. Typically, the casein digest contains a mixture of oligopetides of SEQ ID NO:1, SEQ ID NO:2, SEQ ID NO:3, SEQ ID NO:4, and SEQ ID NO:5 as well as other oligopeptides.

The phosphopeptides may be used as such, or in the form of their alkali metal, alkaline earth metal or transition metal salts. Typical examples are the sodium salts, the calcium salts, the zinc, copper, stannous, potassium, strontium and magnesium salts. Sodium salts are most preferred. The above phosphopeptides are more fully described in EP 268,663.

The method of the present invention is carried out by contacting the teeth with a composition according to the present invention comprising the above-discussed phosphopeptides. Such a composition usually contains from about 0.01 to about 20% by weight of the phosphopeptides, preferably from about 0.1 to about 10%, most preferably from 0.1 to about 5%. Such composition may be an aqueous, aqueous-alcoholic or alcoholic solution or dispersion of the phosphopeptides, e.g. in the form of a mouthwash, or it may be in the form of a dentifrice, a toothpaste, a toothpowder, a gel, a lozenge, a chewing gum or any other suitable form of an oral composition for the care of the teeth.

The other essential element of the present invention is the presence, in the composition which comprises an effective amount of the above-identified phosphopeptides, of an effective amount of a water-soluble polyphosphate salt and/or water-soluble zinc salt. It has been found that these combinations provide for an especially high level of anti-tartar efficacy.

Certain pyrophosphate and other polyphosphate salts have been disclosed in U.S. Patent Nos. 4,515,772 and 4,627,977 as being useful as anti-calculus agents. According to the present invention, preferred polyphosphate salts are inorganic alkalimetal pyrophosphate salts, such as the mono-, di-, tri- and tetraalkalimetal pyrophosphates wherein the alkalimetal is preferably selected from the group consisting of sodium and potassium, and mixtures of these pyrophosphate salts. Polyphosphate salts may be included generally in the amount such that it provides for at least 0.5% polyphosphate anions, the upper level being about 10%, preferably about 7.5%.

Zinc salts are disclosed as anti-calculus and anti-plaque agents in U.S. Patent No. 4,100,269 and in U.S. Patent Nos. 4,416,867, 4,425,325 and 4,339,432. Preferred compositions of the invention include zinc salts, particularly zinc citrate, since it has been found that zinc salts enhance the deposition of phosphopeptides onto saliva coated hydroxyapatite beads. Further, it has been found that zinc decreases the phosphopeptide's loss of activity in an oral environment. When added as zinc sulfate, zinc forms a complex with the phosphopeptide. Addition of zinc as zinc citrate, or concurrent addition of a citrate salt and a zinc salt (i.e. zinc sulfate) prevents formation of the complex. The zinc compounds may be present in the compositions in amounts sufficient to furnish about 0.01% to about 4% zinc, preferably about 0.05% to about 1%, zinc ion.

The preferred oral compositions of the present invention are in the form of toothpaste creams, or gels, or mouthwashes. Ingredients typically included in toothpastes and gels may be used in toothpaste and gel compositions in accordance with the invention. Suitable ingredients include abrasive polishing materials, sudsing agents, flavoring agents, humectants, binders, sweetening agents, and water. Additional anti-calculus agents may be included to enhance the anti-calculus activity of the oral compositions.

Abrasives which may be used in the compositions of the invention include alumina and hydrates thereof, such as alpha alumina trihydrate, magnesium trisilicate, magnesium carbonate, aluminosilicates, such as calcined aluminum silicate and aluminum silicate, calcium carbonate, zirconium silicate, polymethylmethacrylate, powdered polyethylene, silica xerogels, hydrogels and aerogels and the like. Also suitable as abrasive agents are calcium pyrophosphate, insoluble sodium metaphosphate, calcium carbonate, dicalcium orthophosphate, particulate hydroxyapatite and the like. Depending on the form which the oral composition is to take, the abrasive may be present in an amount of from 0 to 70% by weight, preferably 1 to 70% by weight, more preferably from 10 to 70% by weight, particularly for toothpastes.

Humectants contemplated for use in the present invention include glycerol, polyol, sorbitol, polyethylene glycols, propylene glycol, hydrogenated partially hydrolyzed polysaccharides and the like. The humectants are generally present in amounts of from 0 to 80%, preferably 5 to 70% by weight, particularly for toothpastes. Thickeners suitable for use in the invention include silica. Thickeners may be present in toothpaste creams and gels at 0.1 to 20% by weight.

Binders suitable for use in the compositions of the invention include sodium carboxymethyl cellulose, hydroxyethyl cellulose (Natrosol[(R)]) and hydroxypropyl cellulose (Klucel[(R)]), as well as xanthan gums, Irish moss and gum tragacanth. Binders may be present in the toothpaste of the invention to the extent of from 0.01 to 10%. Sweeteners suitable for use in the present dentifrice, preferably at levels of about 0.1% to 5%, include saccharin.

Fluoride sources used in toothpastes such as sodium fluoride, stannous fluoride, sodium monofluorophosphate, zinc ammonium fluoride, tin ammonium fluoride, calcium fluoride and cobalt ammonium fluoride may be, and preferably are, included for delivering anti-caries benefit. It has been found in the present invention that addition of fluoride provides additional stabilization of phosphopeptides. Preferred compositions of the invention include the fluoride source. Fluoride ions are typically provided at a level of from 0 to 1500 ppm, preferably 50 to 1500 ppm, although higher levels up to about 3000 ppm may be used.

Surfactants, such as a soap, anionic, nonionic, cationic, amphoteric and/or zwitterionic, may be present within the range of 0 to 15%, preferably 0.1 to 15%, more preferably 0.25 to 10% by weight. Anionic surfactants are most preferred, such as sodium dodecyl sulfate, sodium lauryl sarcosinate and sodium dodecylbenzene sulfonate.

Flavors are usually included in toothpastes in low amounts, such as from 0.01 to about 5% by weight, especially from 0.1% to 5%.

Ingredients mentioned above as suitable for toothpastes are generally suitable for gels, as will be apparent to one of skill in the art of toothpaste and gel formulation. Thus, except where otherwise noted, references to toothpastes are to be construed as applying to gels as well.

Typically, mouthwashes comprise a water/alcohol solution, flavor, humectant, sweetener, sudsing agent, and colorant. The corresponding compounds mentioned above which are used in toothpastes, are generally suitable within the ranges above for mouthwashes as well. The mouthwash can include ethanol at a level of from 0 to 60%, preferably from 5 to 30% by weight.

Antibacterial agents, for example phenolics such as Triclosan (= 2',4,4'-trichloro-2-hydroxy-diphenylether) (ex Ciba-Geigy) and salicylamides (including salicylanilides), and sources of certain metal ions such as zinc, copper, silver and stannous (e.g., zinc, copper and stannous chloride, and silver nitrate) may also be, and preferably are, included. Coburn et al, U.S. Patent Nos. 4,358,443 and 4,287,191 describe the use of salicylamides and are incorporated by reference herein.

Dyes/colorants suitable for dentifrices, i.e., FD&C Blue #1, FD&C Yellow #10, FD&C Red #40, etc., may be employed in the dentifrices of the invention.

Various other optional ingredients may be included in the compositions of the invention, such as preservatives, vitamins such as vitamin C and E, other anti-plaque agents such as stannous salts, copper salts, strontium salts and magnesium salts. Also included may be pH adjusting agents, anti-caries agents such as urea, calcium glycerophosphate, sodium trimetaphosphate, anti-staining compounds such as silicone polymers, plant extracts, desensitizing agents for sensitive teeth such as potassium nitrate and potassium citrate, and mixtures thereof.

The following specific examples further illustrate the invention, but the invention is not limited thereto.

Preparation of a sodium salt of casein phosphopeptide

A tryptic digest of casein (Hyprol 8052[(R)]) was obtained from Quest International Corporation. A 10% aqueous solution of the digest was prepared and the pH of the resulting solution was adjusted to pH 4.8 by the addition of concentrated hydrochloric acid. The solution was then centrifuged to separate any undigested casein. The supernatant was then made up to 1% calcium chloride, and an equal volume of ethanol was added. The precipitate was collected by centrifugation and freeze dried to yield the dry calcium salt of casein phosphopeptide.

The sodium salt was then prepared by a cation exchange method, as follows.

2 grams of Dowex(R) resin (50WX8, 50-100 mesh, acid form) were added to a 50 ml clear centrifuge tube. The resin was washed three times with 50 ml of water. Subsequently, 40 ml of cold (4°C) 2.5% calcium casein phosphopeptide (clarified by centrifugation) was added to the tube. After gentle agitation in a tube rotator for 30 minutes at 4°C, the acidic supernatant (pH about 2.7) was decanted and neutralized to pH 7.0 with 1.5 ml of 2N NaOH. Calcium content in the supernatant was measured by Atomic Absorption Spectroscopy, and it was found that 99.5% of the original calcium was removed. The resulting solution was freeze-dried to obtain the dry sodium salt of casein phosphopeptide.

The resulting sodium salt of casein phosphopeptide (Na CPP) contained phosphopeptides of SEQ ID NO:1, SEQ ID NO:2 and other casein phosphopeptides. The supernatant obtained prior to the ion exchange step represented phosphopeptide depleted casein peptides.

EXAMPLE 1

Comparative in vitro experiments were carried out using a Seeded Crystal Growth Inhibition Assay. This assay involved the treatment of synthetic hydroxyapatite with the anti-tartar agent to be tested. The hydroxyapatite, treated with the anti-tartar agent was incubated at 37°C in a calcifying solution made up of calcium and phosphate at 1.5 mM and 4.5 mM respectively, pH 7.4. The samples were filtered to remove any calcium phosphate crystals and the free calcium in the filtrate was measured using Atomic Absorption Spectroscopy. The percent inhibition was calculated as follows:

$$\% \text{ Inhibition} = \frac{[Ca] \text{ depletion from control} - [Ca] \text{ depletion from sample}}{[Ca] \text{ depletion from control}}$$

The % inhibition is a measure of the degree of inhibition of calcium phosphate precipitation by the anti-tartar agent. The anti-tartar agents tested were: 1) tetrasodium pyrophosphate, $Na_4 P_2 O_7$ and 2) combinations of $Na_4 P_2 O_7$ and sodium caseinphosphopeptide, Na CPP, prepared as described above, which is a mixture rich in phosphopeptides of SEQ ID NO:1 and SEQ ID NO:2. The following results were obtained:

| $\underline{Na_4 P_2 O_7}$ + | $\underline{\text{Na CPP}}$ | $\underline{\% \text{ Inhibition}}$ |
|---|---|---|
| 0.1 mM | – | 14 |
| " | 0.0075% | 62 |
| " | 0.01% | 68 |
| 0.15 mM | – | 24 |
| " | 0.006% | 64 |
| " | 0.007% | 70 |
| 0.2 mM | – | 54 |
| " | 0.005% | 68 |

EXAMPLE 2

A Microbial Mineralization Assay was carried out. Standard glass rods (3mm x 50mm) were attached to a circular piece of rubber and attached to a vial cap. The surface of the rods was etched. The rods were placed in a growth medium containing sucrose and Streptococcus sobrinus 6715-14. The microbe was grown on the rods for 24 hours at 37°C. The rods were then placed into a fresh growth medium for another 24 hours at 37°C. The rods were then taken out and treated with the anti-tartar agent to be tested. Treatment with only deionized water was used as control. The rods were then placed in a calcifying solution made up of a calcium and phosphate at levels of 1.5 mM and 5.0 mM respectively, at pH 7.4, supplemented with human saliva (about 25%). Six hours later, the treatment with the anti-tartar agent was repeated, and glass rods were placed in a calcifying solution. The procedure was continued for the next three days.

The rods were then removed from the calcifying solution, rinsed with water and placed in 0.6 N HCl solution. The solution containing the rods was heated at 60°C for 2 hours to dissolve calcified salts. The resulting acidic solution was filtered over 0.8 $\mu$m nylon membrane. The level of mineral formation was assessed via calcium and phosphorus analysis. The results were calculated by comparing the % reduction of mineral formation to that of the control.

With 1.5% pyrophosphate anion, 32% reduction was obtained; the combination of 1.5% pyrophosphate anion and 5% Na CPP gave a reduction of 52%.

EXAMPLE 3

Experiments were carried out using a Seeded Crystal Growth Inhibition Assay described in Example 1. % inhibition was measured for NaCPP/Zinc sulphate mixtures. The following results were obtained:

| Agents(s) | Concentration | % Inhibition |
|---|---|---|
| NaCPP/Zinc sulphate | .005%/0.2mM | 0.50 |
| " | .0075%/0.1mM | 0.56 |
| " | .0075%/0.3mM | 0.69 |
| " | .01%/0.1mM | 0.64 |

EXAMPLE 4

Effect of zinc ion addition on the deposition of $C^{14}$-labeled NaCPP onto saliva coated hydroxyapatite beads:

| Zinc concentration (millimolar) | Disintegrations per Minute of Bound NaCPP |
|---|---|
| 0 | 10,815 |
| 5 | 14,232 |
| 15 | 15,621 |

The Example illustrates that the presence of zinc ion enhances the deposition of NaCPP onto saliva coated hydroxyapatite beads.

EXAMPLE 5

Typical formulations for a mouthwash, containing the combination of phosphopeptide and a polyphosphate salt or a zinc salt according to the invention, is as follows:

| MOUTHWASH FORMULATION | | |
|---|---|---|
| INGREDIENTS | % BY WEIGHT OF FINAL COMPOSITION | |
| | A | B |
| Ethanol | 12.5 | 12.5 |
| 70% Sorbitol | 7 | 7 |
| NaCPP | 5 | 5 |
| Tween 20 | 0.55 | 0.55 |
| Preservatives* | 0.2 | 0.2 |
| Flavor | 0.1 | 0.1 |
| Tetrasodium pyrophosphate | 5% | - |
| Zinc citrate trihydrate | - | 1% |
| Dye | <.01 | <.01 |
| Sodium Saccharinate | .065 | .065 |
| Sodium chloride | .05 | .05 |
| Na acetate | .015 | .015 |
| Acetic acid | .015 | .015 |
| $H_2O$ | to 100 | to 100 |
| pH | 6.5 | 6.5 |

\* 0.1% methylparaben
+ 0.1% propylparaben

EXAMPLE 6

Typical toothpaste formulations, containing a phosphopeptide and a polyphosphate salt or a zinc salt according to the invention, are as follows:

| TOOTHPASTE FORMULATIONS pH 6-8 | | | | |
|---|---|---|---|---|
| | % BY WEIGHT OF FINAL COMPOSITION | | | |
| | A | B | C | D |
| 70% Sorbitol | 64 | 39 | 64 | 39 |
| Abrasive silica | 10 | 10 | 10 | 10 |
| Thickening silica | 9 | 10 | 9 | 10 |
| Phosphopeptide (Na salt) | 5 | 5 | 5 | 5 |
| Polyethylene glycol (PEG 32[R]) | 5 | 5 | 5 | 5 |
| Tetrasodium pyrophosphate | 5 | 5 | - | - |
| Zinc citrate trihydrate | - | - | 1 | 1 |
| Sodium Lauryl Sulfate | 1.5 | 1.5 | 1.5 | 1.5 |
| Flavor | 1 | 1 | 1 | 1 |
| Sodium saccharinate | 0.3 | 0.2 | 0.3 | 0.2 |
| Na Fluoride | 0.24 | 0.24 | 0.24 | 0.24 |
| Preservative (Na Benzoate) | .08 | .08 | .08 | .08 |
| Dye | <.01 | --- | <.01 | --- |
| Titanium oxide | --- | 1 | --- | 1 |
| Xanthan Gum | 0.15 | 0.6 | 0.15 | 0.6 |
| $H_2O$ | to 100 | to 100 | to 100 | to 100 |

## Claims

1. The method of controlling dental tartar which comprises treating the teeth in the mouth with an oral composition which comprises an effective amount of a phosphopeptide or a salt thereof, the phosphopeptide having from 5 to 30 aminoacids including the aminoacid sequence A-B-C-D-E, wherein A, B, C, D and E are independently phosphoserine, phosphothreonine, phosphotyrosine, phosphohistidine, glutamate and aspartate, and an effective amount of an anti-calculus agent selected from the group consisting of water-soluble polyphosphate salts, water-soluble zinc salts and mixtures thereof.

2. The method according to claim 1, wherein the phosphopeptide comprises the sequence A-B-C-D-E wherein A, B and C are each phosphoserine, and D an E are each glutamate.

3. The according to claim 2, wherein the phosphopeptide comprises a mixture of phosphopeptides of SEQ ID NO:1 and SEQ ID NO:2.

4. The method according to claim 1, wherein the composition is applied in the form of an aqueous mouth rinse.

5. The method according to claim 1, wherein the composition is applied in the form of a toothpaste.

15

6. The method according to claim 1 wherein the phosphopeptide is a derivative of a naturally occurring material.

7. The method according to claim 6 wherein the naturally occurring material is a casein.

8. An oral composition, suitable for controlling dental tartar, comprising an effective amount of a phosphopeptide or a salt thereof, the phosphopeptide having from 5 to 30 aminoacids including the aminoacid sequence A-B-C-D-E, wherein A, B, C, D and E are independently phosphoserine, phosphothreonine, phosphotyrosine, phosphohistidine, glutamate and aspartate, and a water-soluble polyphosphate salt in an amount equivalent to at least 0.5% by weight of polyphosphate anions.

9. The oral composition according to claim 8, wherein the phosphopeptide comprises the sequence A-B-C-D-E wherein A, B and C are each phosphoserine, and D an E are each glutamate.

10. The oral composition according to claim 9 wherein the phosphopeptide comprises a mixture of phosphopeptides having SEQ ID NO:1 and SEQ ID NO:2.

11. The oral composition according to claim 8, comprising from 0.01% - 10% by weight of the phosphopeptide.

12. The oral composition according to claim 8 wherein the polyphosphate salt is an alkalimetal pyrophosphate salt.

13. The oral composition according to claim 8 wherein the phosphopeptide is a derivative of a naturally occurring material.

14. The oral composition according to claim 13 wherein the naturally occurring material is a casein.

15. An oral composition, suitable for controlling dental tartar, comprising an effective amount of a phosphopeptide or a salt thereof, the phosphopeptide having from 5 to 30 aminoacids including the aminoacid sequence A-B-C-D-E, wherein A, B, C, D and E are independently phosphoserine, phosphothreonine, phosphotyrosine, phosphohistidine, glutamate and aspartate, and a water-soluble zinc salt in an amount effective to control tartar.

16. The composition according to claim 15 wherein the zinc salt is present in an amount sufficient to furnish about 0.05% to about 4% zinc ion.

17. The composition according to claim 15 wherein the zinc salt is zinc citrate.

18. The composition according to claim 15 wherein the phosphopeptide comprises the sequence A-B-C-D-E wherein A, B and C are each phosphoserine, and D an E are each glutamate.

19. The composition according to claim 18 wherein the phosphopeptide comprises a mixture of phosphopeptides of SEQ ID NO:1 and SEQ ID NO:2.

20. The composition according to claim 15 wherein the phosphopeptide is a derivative of a derivative of a naturally occurring material.

21. The composition according to claim 20 wherein the naturally occurring material is a casein.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X<br>Y<br>D | WO-A-8 707 615 (THE UNIVERSITY OF MELBOURNE ET AL.)<br>* claims 1-28; example 20 *<br>& EP-A-0 268 663 | 15-16, 18-21<br>1-14 | A61K7/16 |
| Y | PATENT ABSTRACTS OF JAPAN vol. 13, no. 417 (C-636)(3765) 14 September 1989<br>& JP-A-01 153 621 ( SUNSTAR INC ) 15 June 1989<br>* abstract *<br>* the whole document * | 1-14 | |
| P,A | WO-A-9 113 607 (HENKEL)<br>* page 3, paragraph 3 *<br>* page 6, line 1 - line 5; claims 1-4 * | 1-21 | |
| A<br>D | EP-A-0 297 213 (THE PROCTER & GAMBLE CO)<br>* claims 1-6 *<br>& US-A-4 515 772 | 1-21 | |
| | | | **TECHNICAL FIELDS SEARCHED (Int. Cl.5 )** |
| | | | A61K |

The present search report has been drawn up for all claims

| Place of search | Date of completion of the search | Examiner |
|---|---|---|
| BERLIN | 17 NOVEMBER 1992 | SIATOU E. |

EPO FORM 1503 03.82 (P0401)

## Sequence Listing

(1) GENERAL INFORMATION:

   (i) APPLICANT: Burger, Allan R.

   (ii) TITLE OF INVENTION: Method of Controlling Dental Tartar and Compositions for Use Therein.

   (iii) NUMBER OF SEQUENCES: 5

   (iv) CORRESPONDENCE ADDRESS:

      (A) ADDRESSEE: Patent Department, Unilever United States, Inc.

      (B) STREET: 45 River Road

      (C) CITY: Edgewater

      (D) STATE: New Jersey

      (E) COUNTRY: USA

      (F) ZIP: 07020

   (v) COMPUTER READABLE FORM:

      (A) MEDIUM TYPE: Diskette-3.5 inch, 1.44 Mb storage

      (B) COMPUTER: IBM PS/2

      (C) OPERATING SYSTEM: MS-DOS

      (D) SOFTWARE: Microsoft Word$^R$

(2) INFORMATION FOR SEQ. ID NO:1:

   (i) SEQUENCE CHARACTERISTICS:

      (A) LENGTH: 21

      (B) TYPE: Amino acid

(ix) FEATURE:

    (A) NAME/KEY: Phosphoserine

    (B) LOCATION: 6

    (D) OTHER INFORMATION: Post-translationally phosphorylated serine

(ix) FEATURE:

    (A) NAME/KEY: Phosphoserine

    (B) LOCATION: 8

    (D) OTHER INFORMATION: Post-translationally phosphorylated serine

(ix) FEATURE:

    (A) NAME/KEY: Phosphoserine

    (B) LOCATION: 9

    (D) OTHER INFORMATION: Post-translationally phosphorylated serine

(ix) FEATURE:

    (A) NAME/KEY: Phosphoserine

    (B) LOCATION: 10

    (D) OTHER INFORMATION: Post-translationally phosphorylated serine

(ix) FEATURE:

    (A) NAME/KEY: Phosphoserine

    (B) LOCATION: 17

    (D) OTHER INFORMATION: Post-translationally phosphorylated serine

(xi) SEQUENCE DESCRIPTION: SEQ ID NO:1:

Glu Met Glu Ala Glu Ser Ile Ser Ser Ser Glu Glu Ile Val Pro Asn

1                    5                         10                        15

Ser Val Glu Gln Lys

          20

(2) INFORMATION FOR SEQ. ID NO:2:

 (i) SEQUENCE CHARACTERISTICS:

  (A) LENGTH: 24

  (B) TYPE: Amino acid

  (C) STRANDEDNESS:

  (D) TOPOLOGY:

 (ix) FEATURE:

  (A) NAME/KEY: Phosphoserine

  (B) LOCATION: 14

  (D) OTHER INFORMATION: Post-translationally phosphorylated serine

 (ix) FEATURE:

  (A) NAME/KEY: Phosphoserine

  (B) LOCATION: 16

  (D) OTHER INFORMATION: Post-translationally phosphorylated serine

 (ix) FEATURE:

  (A) NAME/KEY: Phosphoserine

  (B) LOCATION: 17

  (D) OTHER INFORMATION: Post-translationally phosphorylated serine

(ix) FEATURE:

    (A) NAME/KEY: Phosphoserine

    (B) LOCATION: 18

    (D) OTHER INFORMATION: Post-translationally phosphorylated

       serine

(xi) SEQUENCE DESCRIPTION: SEQ ID NO:2:

Glu Leu Glu Glu Leu Asn Val Pro Gly Glu Ile Val Glu Ser Leu Ser

1             5             10            15

Ser Ser Glu Glu Ser Ile Thr Arg

       20

(2) INFORMATION FOR SEQ. ID NO:3:

    (i) SEQUENCE CHARACTERISTICS:

       (A) LENGTH: 20

       (B) TYPE: Amino acid

    (ix) FEATURE:

       (A) NAME/KEY: Phosphoserine

       (B) LOCATION: 7

       (D) OTHER INFORMATION: Post-translationally phosphorylated

         serine

    (ix) FEATURE:

       (A) NAME/KEY: Phosphoserine

       (B) LOCATION: 8

       (D) OTHER INFORMATION: Post-translationally phosphorylated

         serine

    (ix) FEATURE:

        (A) NAME/KEY: Phosphoserine

        (B) LOCATION: 9

        (D) OTHER INFORMATION: Post-translationally phosphorylated

            serine

    (ix) FEATURE:

        (A) NAME/KEY: Phosphoserine

        (B) LOCATION: 15

        (D) OTHER INFORMATION: Post-translationally phosphorylated

            serine

    (xi) SEQUENCE DESCRIPTION: SEQ ID NO:3:


Asn Thr Met Glu His Val Ser Ser Ser Glu Glu Ser Ile Ile Ser Gln

1                   5                   10                  15


Glu Thr Tyr Lys

                    20


    (2) INFORMATION FOR SEQ. ID NO:4:

        (i) SEQUENCE CHARACTERISTICS:

            (A) LENGTH: 25

            (B) TYPE: Amino acid

        (ix) FEATURE:

            (A) NAME/KEY: Phosphoserine

            (B) LOCATION: 11

            (D) OTHER INFORMATION: Post-translationally phosphorylated

                serine

```
(ix) FEATURE:

    (A) NAME/KEY: Phosphoserine

    (B) LOCATION: 12

    (D) OTHER INFORMATION: Post-translationally phosphorylated

        serine

(ix) FEATURE:

    (A) NAME/KEY: Phosphoserine

    (B) LOCATION: 13

    (D) OTHER INFORMATION: Post-translationally phosphorylated

        serine

(ix) FEATURE:

    (A) NAME/KEY: Phosphoserine

    (B) LOCATION: 16

    (D) OTHER INFORMATION: Post-translationally phosphorylated

        serine

(xi) SEQUENCE DESCRIPTION: SEQ ID NO:4:


Asn Ala Asn Glu Glu Glu Tys Ser Ile Gly Ser Ser Ser Glu Glu Ser
1               5                   10                  15


Ala Glu Val Ala Thr Glu Glu Val Lys
            20                  25


(2) INFORMATION FOR SEQ. ID NO:5:

    (i) SEQUENCE CHARACTERISTICS:

        (A) LENGTH: 11

        (B) TYPE: Amino acid
```

```
(ix) FEATURE:

     (A) NAME/KEY: Phosphoserine

     (B) LOCATION: 4

     (D) OTHER INFORMATION: Post-translationally phosphorylated

         serine

(ix) FEATURE:

     (A) NAME/KEY: Phosphothreonine

     (B) LOCATION: 5

     (D) OTHER INFORMATION: Post-translationally phosphorylated

         threonine

(ix) FEATURE:

     (A) NAME/KEY: Phosphoserine

     (B) LOCATION: 6

     (D) OTHER INFORMATION: Post-translationally phosphorylated

         serine

(xi) SEQUENCE DESCRIPTION: SEQ ID NO:5:


Glu Gln Leu Ser Thr Ser Glu Glu Asn Ser Lys

1               5                   10
```